**Europäisches Patentamt**

**European Patent Office**

**Office européen des brevets**

(19)

(11) Numéro de publication: **0 110 793**
**B1**

(12) # FASCICULE DE BREVET EUROPÉEN

(45) Date de publication du fascicule du brevet:
**18.03.87**

(21) Numéro de dépôt: **83402330.1**

(22) Date de dépôt: **02.12.83**

(51) Int. Cl.⁴: **A 61 F 13/20**

(54) **Tampon périodique aseptique.**

(30) Priorité: **02.12.82 FR 8220216**

(43) Date de publication de la demande:
**13.06.84 Bulletin 84/24**

(45) Mention de la délivrance du brevet:
**18.03.87 Bulletin 87/12**

(84) Etats contractants désignés:
**AT BE DE GB IT NL**

(56) Documents cité:
**US-A-1 575 123**
**US-A-2 739 593**
**US-A-4 317 447**

(73) Titulaire: **Pasquini, Michel, 4 rue Antoine Despont,
F-24170 Belves (FR)**

(72) Inventeur: **Pasquini, Michel, 4 rue Antoine
Despont, F-24170 Belves (FR)**

(74) Mandataire: **Martinet & Lapoux, 62, rue des
Mathurins, F-75008 Paris (FR)**

## Description

La présente invention concerne des perfectionnements aux tampons périodiques dits également tampons cataméniaux et, plus particulièrement, des tampons périodiques ayant des propriétés d'antisepsie.

Le changement d'un tampon usagé par un tampon neuf s'effectue avec des intervalles de temps variables qui dépendent du débit du flux périodique et de la capacité d'absorption des tampons. Chaque remplacement est susceptible d'introduire dans le milieu vaginal des germes banaux, plus ou moins pathogènes, présents au niveau de la vulve et sur la surface cutanée adjacente. Or, ce milieu est périodiquement hautement favorable au développement rapide des germes dont il est impossible de prévoir la pathogénicité.

Le défaut d'antisepsie suffisante des tampons périodiques est confirmé par la clinique et la pratique médicale quotidienne qui montrent de nombreux cas de vaginites récidivantes à germes banaux ou spécifiques (souvent à candida albicans) induites ou aggravées par l'emploi intempestif de tampons incapables d'assurer l'antisepsie du milieu dans lequel ils assurent leur fonction.

On connaît par US-A-1575123 des tampons composés d'un conteneur en forme de capsule en matériau dissolvable, en gélatine par exemple, ledit container étant rempli d'un tampon absorbant proprement dit. L'extrémité ouverte du conteneur est fermée par un couvercle. L'autre extrémité du conteneur opposé au couvercle a été pressée par construction vers l'intérieur de façon à présenter une partie concave, alvéole ou cupule, la face de cette partie correspondant de façon générale au col utérin.

L'objet de la présente invention est de procurer des tampons périodiques antiseptiques.

Le tampon périodique de l'invention comprend un tampon absorbant comportant un alvéole en forme de cupule à l'extrémité dudit tampon, et il est caractérisé en ce qu'il comprend en outre un embout rond relativement plat fixé dans ledit alvéole par une gomme adhésive soluble, ledit embout étant de la nature des ovules gynécologiques.

Le tampon peut en outre comprendre des saignées longitudinales à la surface extérieure latérale du tampon et dans ces saignées des capsules de forme appropriée contenant la même matière médicamenteuse que l'embout ou une matière médicamenteuse différente.

L'invention va être maintenant décrite en détail, en relation avec les dessins annexés dans lesquels:

- les Figs. 1a et 1b représentent respectivement, de champ et en bout, un tampon périodique de l'art antérieur;

- la Fig. 2 représente un tampon périodique du type des Figs. 1a et 1b, ayant une section terminale préformée en alvéole pour recevoir une capsule conformément à l'invention;

- la Fig. 3 représente un tampon périodique muni de sa capsule conformément à l'invention;

- la Fig. 4 représente un tampon du type des Figs. 1a et 1b dans lequel l'embout est creusé en alvéole et les saignées latérales sont approfondies; et

- la Fig. 5 représente le tampon muni de ses capsules terminale et latérales.

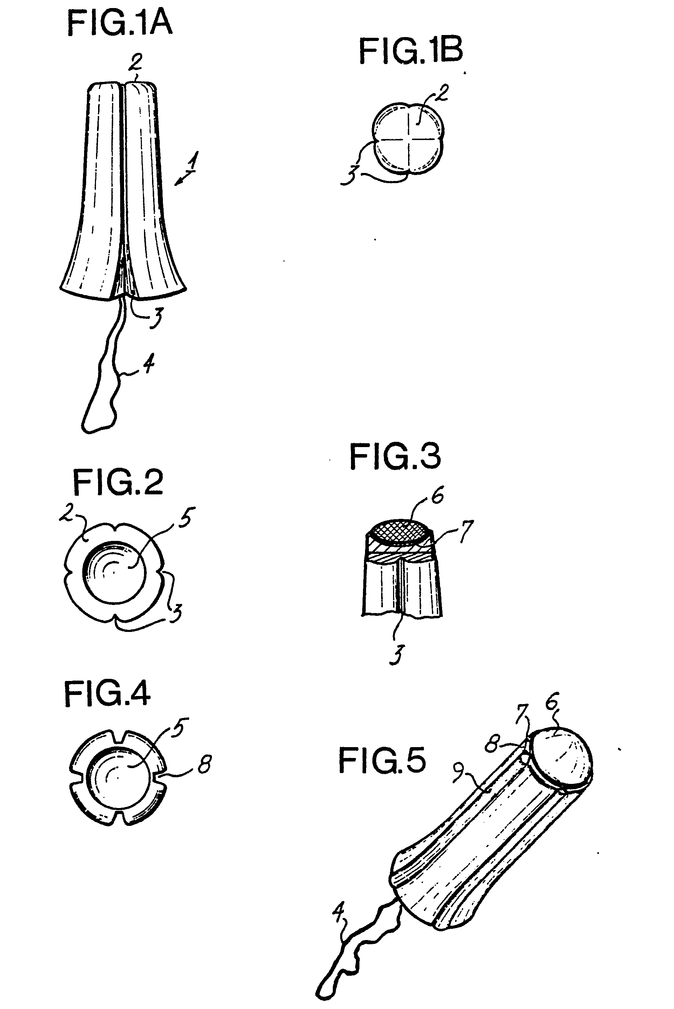

Les Figs. 1a et 1b représentent un tampon périodique d'un type courant. Le numéro de référence 1 désigne le tampon, 2 sa face terminale, 3 quatre saignées longitudinales peu profondes et 4 le cordon de retrait. La longueur du tampon périodique est sensiblement de 50mm.

Dans la Fig. 2, le tampon comprend un alvéole terminal 5 en forme de cupule.

Dans la Fig. 3, un ovule gynécologique 6 est placé dans l'alvéole 5 et y est maintenu par une gomme adhésive formée d'un excipient soluble. La couche de gomme est représentée en 7.

La Fig. 4 représente un tampon ayant un alvéole terminal 5 et des saignées approfondies 8 et la Fig. 5 représente un tampon conforme à une seconde réalisation de l'invention. Un ovule 6 est logé dans l'alvéole 5 et des capsules 9 sont logées dans les saignées 8. L'ovule 6 et les capsules 9 sont maintenues solidaires du tampon par une gomme adhésive soluble 7.

Le contenu de l'ovule et des capsules latérales peut être l'une des compositions couramment employées dans les traitements gynécologiques.

Premier exemple

| Formule | par capsule |
|---|---|
| Sulfate de Néomycine | 6.000 U |
| Sulfate de Polymyxine B | 6.000 U |
| Nystatine | 16.000 U |
| Acétarsol | 0,025 g |
| Sorbate de Potassium | 0,2 g |
| Excipient | |

Deuxième exemple

| | |
|---|---|
| Ternidazole (DCI) | 50 mg |
| Néomycine (DCI) sulfate | 25 mg |
| soit | 16.000 U |
| Nystatine (DCI) | 25.000 U |
| Prednisolone (DCI) sous forme de métasulfobenzoate | |
| sodique 1 mg | |

+ excipient comprenant: amidon, lactose, benzoate, silice, talc, stéarate de magnésium, laurylsulfate de sodium, polyvirone-excipient, essences de géranium et de girofle.

Troisième exemple

| | |
|---|---|
| Promestriène (DCI) | 3 mg |
| Chloroquinaldol (DCI) | 70 mg |
| Excipient Acide Borique | 25 mg |
| Laurylsulfate de sodium | 0,5 mg |
| Stéarate de magnésium | |

La gomme adhésive soluble peut être la gomme connue sous le nom de KARAYA (Cf."DORVAULT" Officine Répertoire Général de Pharmacie Pratique) 18ième Edition - Bis - page 988.

D'une façon générale la composition médicamenteuse de l'ovule et des capsules doit avoir à la fois une action antibactérienne, une action antimycosique et une action antiparasitaire notamment contre le trichomonas.

Les avantages des tampons conformes à l'invention sont les suivants:

Les tampons de l'art antérieur ne sont modifiés ni quant à leur forme ni quant à leur longueur et leur diamètre.

La fixation de l'ovule et des capsules par application d'une gomme neutre adhésive et soluble ne complique pas le procédé de fabrication des tampons.

Le contenu de l'ovule se délite d'une manière homogène dans le flux périodique ce qui permet une action antiseptique efficace et constante sur toutes les faces du tampon et sur toute la longueur du conduit vaginal. L'effet antiseptique se trouve naturellement renforcé au niveau du col utérin qui est souvent le siège d'infections chroniques ou récidivantes aux conséquences souvent graves.

**Revendications**

1 - Tampon périodique antiseptique comprenant un tampon absorbant comportant un alvéole en forme de cupule (5) à l'une de ses extrémités, caractérisé en ce qu'il comprend en outre un ovule rond relativement plat (6) fixé dans ledit alvéole par une gomme adhésive soluble, ledit ovule étant rempli d'une matière médicamenteuse ou antiseptique.

2 - Tampon périodique antiseptique conforme à la revendication 1, caractérisé en ce qu'il comprend en outre des saignées longitudinales (8) et dans ces saignées des capsules allongées (9) contenant une matière médicamenteuses ou antiseptique.

**Patentansprüche**

1. Aseptischer Menstruations-Tampon mit einer becherförmigen Kammer an dem einen Ende des saugfähigen Tampons, dadurch gekennzeichnet, daß

- in der becherförmigen Kammer (5) ein runder abgeflachter Eikörper (6) mittels einer löslichen Klebschicht (7) befestigt ist,

- der ein arzneiliches oder aseptisches Mittel enthält.

2. Aseptischer Menstruations-Tampon nach Anspruch 1, dadurch gekennzeichnet, daß

- zusätzliche längsverlaufende Schlitze (8) vorgesehen sind,

- in denen langgestreckte Kapseln (9) mit einem arzneilichen oder aseptischen Mittel untergebracht sind.

**Claims**

1 - Antiseptic periodic tampon including an absorbing tampon comprising a cupule shaped alveolus (5) at one end of the said tampon, characterised in that it further comprises a substantially flat rounded ovule (6) bounded to said alveolus by means vt a soluble adhesive gum, said ovule being filled with a medicamentous or antiseptic material.

2 - Antiseptic periodic tampon according to claim 1, characterised in that it further comprises longitudinal grooves (8) and in these grooves elongated capsules (9) containing a medicamentous or antiseptic material.

0 110 793

## FIG.1A

2

1

3

4

## FIG.1B

2

3

## FIG.2

2

5

3

## FIG.3

6

7

3

## FIG.4

5

8

## FIG.5

7

6

8

9

4

1